(19) Europäisches Patentamt
European Patent Office
Office européen des brevets

(11) **EP 1 832 227 A1**

(12) **DEMANDE DE BREVET EUROPEEN**

(43) Date de publication:
**12.09.2007 Bulletin 2007/37**

(51) Int Cl.:
*A61B 5/0245* (2006.01)

(21) Numéro de dépôt: **06110828.8**

(22) Date de dépôt: **08.03.2006**

| | |
|---|---|
| (84) Etats contractants désignés:<br>**AT BE BG CH CY CZ DE DK EE ES FI FR GB GR HU IE IS IT LI LT LU LV MC NL PL PT RO SE SI SK TR**<br>Etats d'extension désignés:<br>**AL BA HR MK YU**<br><br>(71) Demandeur: **EM Microelectronic-Marin SA**<br>**2074 Marin (CH)** | (72) Inventeur: **Novac, Pinchas**<br>**2000, Neuchâtel (CH)**<br><br>(74) Mandataire: **Vigand, Philippe et al**<br>**ICB,**<br>**Ingénieurs Conseils en Brevets SA,**<br>**Rue des Sors 7**<br>**2074 Marin (CH)** |

(54) **Circuit de conditionnement du signal entre un dispositif optique et une unité de traitement**

(57) L'invention concerne un circuit de conditionnement (10) d'un signal externe (IN) représentatif d'une grandeur physiologique, agencé entre un capteur optique (11) et une unité de traitement (12), le signal externe (IN) reçu étant décomposé en une composante utile et une composante ambiante, caractérisé en ce que le circuit de conditionnement comprend un premier étage (13) comprenant un amplificateur de transimpédance avec un filtre passe-haut (15) incorporé utilisant une boucle de rétroaction pour soustraire au signal externe reçu, la composante ambiante du signal et délivrer en sortie un signal utile amplifié (IN1), un deuxième étage (16) comprenant un circuit échantillonneur bloqueur (17) pour démoduler le signal utile amplifié et délivrant en sortie un signal utile démodulé (IN2), et un troisième étage (18) comprenant un filtre passe-bande (19) pour filtrer le signal utile démodulé dans la bande de fréquence de la grandeur physiologique à détecter et pour transmettre un signal conditionné (OUT) à l'unité de traitement.

Fig.1

EP 1 832 227 A1

## Description

DOMAINE TECHNIQUE

La présente invention concerne, de manière générale, un circuit de conditionnement du signal entre un capteur optique pour la mesure d'une grandeur physiologique, notamment du rythme cardiaque, et une unité de traitement numérique des signaux reçus. L'invention concerne plus particulièrement les différents étages de ce circuit de conditionnement, en particulier, les étages d'amplification, d'échantillonnage blocage, et de filtrage. L'invention concerne également un circuit intégré comprenant un tel circuit de conditionnement. L'invention concerne encore un instrument électronique portable comprenant un tel circuit intégré.

ARRIERE-PLAN TECHNOLOGIQUE

Il est connu dans l'art antérieur, en particulier du document EP 1 484 009, un instrument portable muni d'un dispositif optique de mesure d'une grandeur physiologique. Ce document décrit en particulier, comme représenté à la figure 6, un instrument portable comportant une source lumineuse 61 (par exemple une diode électroluminescente, i.e. LED, ou tout autre dispositif adéquat) couplée à un circuit de commande 71 dont le fonctionnement est contrôlé par une unité centrale de traitement 70, tel un microprocesseur ou microcontrôleur. Cette unité centrale 70 est par ailleurs interfacée avec un dispositif d'affichage 73 (de type analogique et/ou digital), des moyens de mémorisation 74 (RAM, ROM, EEPROM, FLASH ou analogues) et une horloge système 75 assurant le cadencement adéquat du fonctionnement de l'unité centrale 70 et de ses composants périphériques. Cette horloge système 75 peut par ailleurs assurer les fonctions horaires classiques d'un garde-temps.

L'unité centrale de traitement est encore couplée à un circuit 72 dédié à la détection de la mesure de la grandeur physiologique désirée, par exemple le rythme cardiaque ou le taux d'oxygène dans le sang, les fonctions de ce circuit pouvant être intégrées avec celles de l'unité centrale de traitement 70. Ce circuit permet d'extraire les informations relatives à la grandeur physiologique à partir des signaux optiques détectés par le ou les photorécepteurs associés. Dans le cas d'espèce, un premier photorécepteur 62 est couplé au circuit de détection 72 via un moyen d'amplification et, le cas échéant de filtrage 63. Les informations relatives à la grandeur physiologique désirée sont transmises à l'unité centrale de traitement 70, notamment dans le but d'être affichées sur le dispositif 73 et/ou stockées dans les moyens de mémorisation 74 pour une consultation ultérieure.

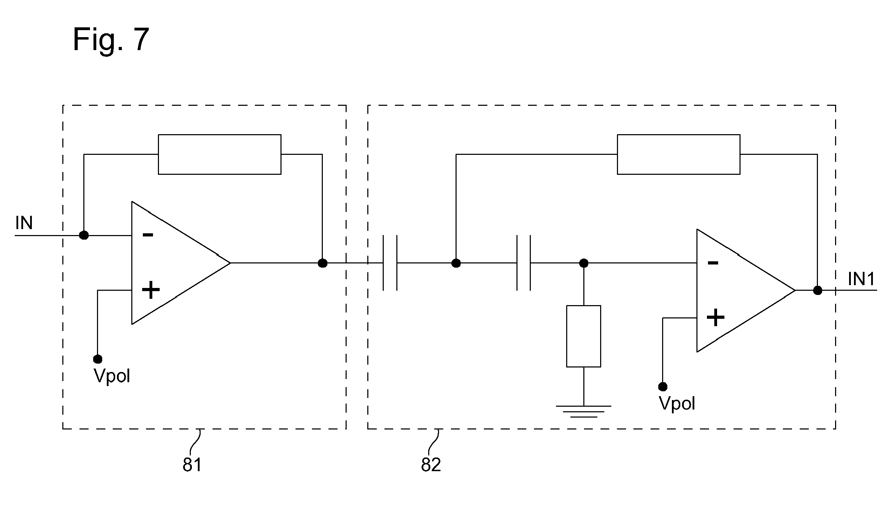

Selon un mode de réalisation envisageable, représentée à la figure 7, il est prévu pour réaliser les moyens d'amplification et de filtrage en sortie du photorécepteur, un amplificateur en série d'un filtre passe-haut. Le signal externe IN est tout d'abord amplifié au travers d'un circuit amplificateur 81, assurant une fonction de convertisseur courant tension. Le premier étage comprend en outre du circuit amplificateur 81, un filtre passe-haut 82 pouvant être réalisé par exemple au moyen d'un filtre passe-haut à un amplificateur à gain fini du type Sallen et Key. Ainsi, la composante ambiante du signal externe IN, qui n'est pas modulée, est supprimée au travers du filtre passe-haut 82. Un signal IN1 amplifié, comprenant que la composante utile du signal détecté, est transmis en sortie de ces moyens d'amplification et de filtrage à l'étage suivant pour le conditionnement de ces signaux.

Néanmoins, dans le cadre de la présente invention, il a été mis en évidence qu'une telle solution n'est pas optimale dans la mesure où le signal ambiant composante du signal externe IN reçu est également amplifié au travers de l'amplificateur 81, ce qui limite passablement la plage de gain utilisable de cet amplificateur pour éviter que ce dernier sature. Le rapport signal sur bruit serait alors moins favorable à l'entrée de l'étage suivant.

De plus, un tel instrument portable et plus particulièrement le circuit de conditionnement permettant de conditionner les signaux reçus par le capteur optique avant leur traitement par l'unité centrale de traitement, présente certains inconvénients notamment en terme de place occupée dans l'instrument portable et en terme de consommation. En effet, dans un tel instrument électronique portable, deux soucis majeurs sont typiquement l'espace disponible et la consommation d'énergie qui sont tous deux limités. Un circuit de conditionnement tel que présenté dans l'art antérieur présente une surface occupée non optimale dans la mesure où il utilise un certain nombre de composants discrets pour réaliser les fonctions d'amplification, de filtrage et de détection. De plus, chacune de ces fonctions présente une consommation d'énergie non optimisée en raison notamment de l'utilisation de nombreux amplificateurs opérationnels.

RESUME DE L'INVENTION

L'un des buts principaux de la présente invention est de pallier aux inconvénients susmentionnés en implémentant un circuit de conditionnement entre un capteur optique et une unité de traitement dont au moins une partie des éléments ont été intégrées et dont la consommation a été optimisée.

A cet effet, la présente invention concerne un circuit de conditionnement d'un signal externe représentatif d'une grandeur physiologique entre un capteur optique et une unité de traitement, le signal externe reçu étant décomposé en une composante utile et une composante ambiante, caractérisé en ce que le circuit de conditionnement comprend un premier étage comprenant un amplificateur de transimpédance avec un filtre passe-haut incorporé utilisant une boucle de rétroaction pour soustraire, en entrée de l'étage, au signal externe reçu, la composante ambiante du signal et délivrer en sortie un signal utile amplifié, un deuxième étage comprenant un circuit échantillonneur bloqueur pour démoduler le signal utile amplifié et délivrant en sortie un signal utile démodulé, et un troisième étage comprenant un filtre passe-bande pour filtrer le signal utile démodulé dans la bande de fréquence de la grandeur physiologique à détecter et pour transmettre un signal conditionné à l'unité de traitement.

Des variantes avantageuses de ce circuit de conditionnement sont données en relation avec les revendications dépendantes.

L'invention concerne aussi un circuit intégré comprenant un circuit de conditionnement selon l'invention.

Enfin la présente invention concerne encore un instrument électronique portable au poignet comprenant un dispositif optique pour la mesure d'une grandeur physiologique, notamment du rythme cardiaque, le dispositif optique comprenant au moins une source lumineuse pour soumettre une portion d'un tissu organique à une émission lumineuse et au moins un capteur optique pour détecter l'intensité de l'émission lumineuse après propagation dans le tissu organique, un circuit intégré comprenant un circuit de conditionnement selon l'invention et agencé pour traiter les signaux optiques détectés par le capteur optique, et un dispositif d'affichage pour afficher une information relative à la mesure de la grandeur physiologique, notamment le rythme cardiaque.

BREVE DESCRIPTION DES FIGURES

D'autres caractéristiques et avantages de la présente invention apparaîtront plus clairement à la lecture de la description détaillée qui suit de modes de réalisation de l'invention donnés uniquement à titre d'exemple non limitatif et illustrés par les dessins annexés où :

- la figure 1 est un schéma bloc fonctionnel montrant l'ensemble du circuit de conditionnement selon un mode de réalisation de l'invention;
- la figure 2a est un schéma fonctionnel d'un mode de réalisation avantageux du premier étage du circuit de conditionnement selon l'invention;
- la figure 2b représente un exemple d'implémentation préféré du mode de réalisation du premier étage de la figure 2a;
- la figure 3a est un schéma fonctionnel selon un mode de réalisation du deuxième étage du circuit de conditionnement de l'invention;
- la figure 3b est un schéma fonctionnel selon un autre mode de réalisation du deuxième étage du circuit de conditionnement de l'invention;
- la figure 3c représente un exemple d'implémentation préféré du deuxième étage du circuit de conditionnement;
- la figure 3d représente les signaux de commande appliqués au niveau du deuxième étage selon les exemples de réalisation des figures 3b et 3c;

- la figure 4a est un schéma fonctionnel selon un mode de réalisation du troisième étage du circuit de conditionnement selon l'invention;
- la figure 4b représente la première section d'un filtre passe-bande d'ordre 5 du troisième étage du circuit de conditionnement;
- la figure 4c représente la deuxième section d'un filtre passe-bande d'ordre 5 du troisième étage du circuit de conditionnement;
- la figure 4d représente un circuit de remplacement pour chacun des quatre premiers amplificateurs opérationnels des figures 4b et 4c;
- la figure 5 est une vue en coupe d'un instrument électronique portable comprenant un circuit de conditionnement selon un mode de réalisation de l'invention;
- la figure 6, déjà décrite, est un schéma bloc illustrant divers composants d'un instrument portable selon l'art antérieur;
- la figure 7 est un schéma fonctionnel d'un mode de réalisation du premier étage du circuit de conditionnement.

DESCRIPTION DETAILLEE DE L'INVENTION

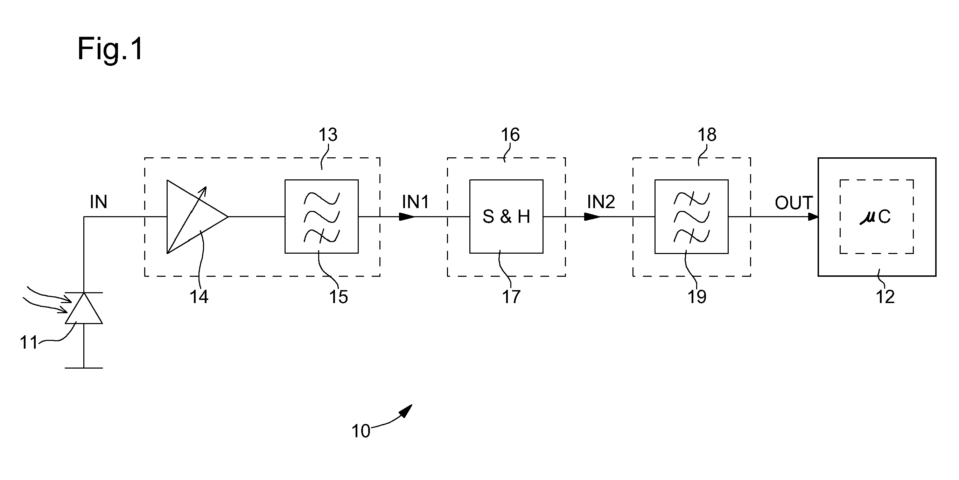

Les divers modes de réalisation qui vont maintenant être présentés sont donnés à titre purement illustratif et non limitatif. La figure 1 est un schéma bloc montrant l'ensemble du circuit de conditionnement 10 selon un mode de réalisation de l'invention. Pour des raisons de compréhensions générales, il a été également représenté sur cette figure 1, un capteur optique 11, comme par exemple une photodiode, un phototransistor ou tout autre récepteur optique adéquat recevant un signal externe IN permettant la mesure d'une grandeur physiologique, notamment du rythme cardiaque. Ce signal externe IN est un signal de courant comprenant une composante ambiante et une composante utile, ledit signal étant obtenu par exemple au moyen d'un dispositif optique comprenant au moins une source lumineuse (non représentée) pour soumettre une portion d'un tissu organique à une émission lumineuse et au moins un photorécepteur, i.e. le capteur optique 11, pour détecter l'intensité de l'émission lumineuse après propagation dans le tissu organique. Il a également été représenté une unité centrale de traitement 12, tel un microprocesseur ou microcontrôleur recevant en entrée, au moyen d'un convertisseur analogique numérique, le signal externe conditionné OUT au travers du circuit de conditionnement 10 permettant d'effectuer un traitement des signaux reçus.

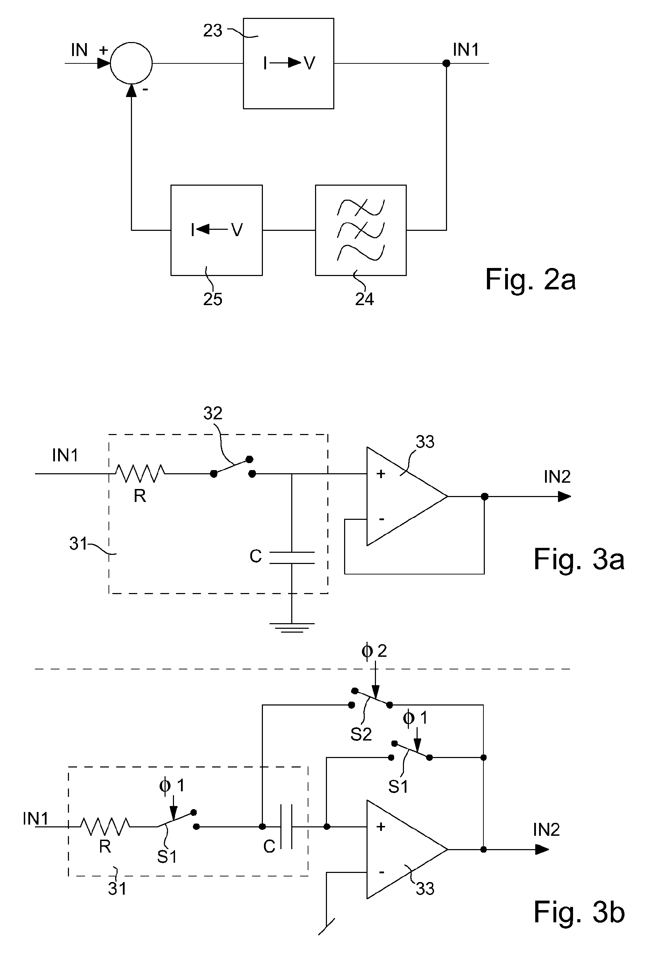

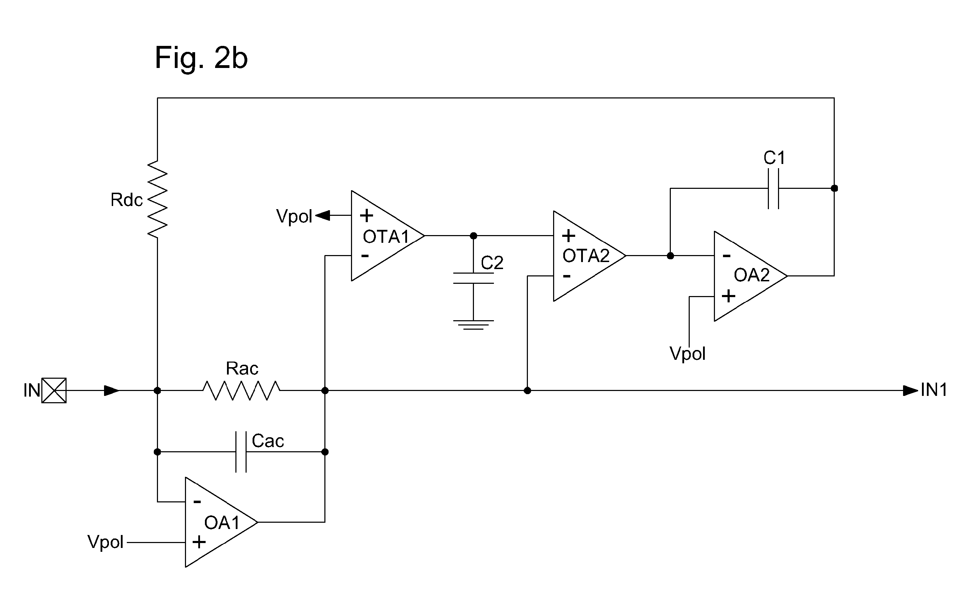

Le circuit de conditionnement 10 selon l'invention permet de réaliser ce traitement tout en optimisant la consommation par rapport aux solutions traditionnelles. Ce circuit de conditionnement comprend notamment un premier étage 13 amplificateur de transimpédance pour pré-amplifier le signal externe IN et convertir le courant détecté en une tension. Le signal est ensuite filtré pour éliminer les composantes basses fréquences non modulées. Généralement pour cela, le signal externe IN est tout d'abord amplifié au travers d'un circuit amplificateur 14, assurant une fonction de convertisseur courant tension, puis filtré au moyen d'un filtre passe-haut 15 pouvant être réalisé par exemple au moyen d'un filtre passe-haut à un amplificateur à gain fini du type Sallen et Key. En vue de pallier aux inconvénients d'un premier étage tel que présenté en relation avec la figure 7, des variantes avantageuses de réalisation du premier étage 13, présentées plus en détail en relation avec les figures 2a et 2b, comprennent une boucle de rétroaction permettant de n'amplifier que la composante utile du signal IN, i.e. amplitude des pulsations détectées, après filtrage de la composante ambiante du signal par soustraction via la boucle de rétroaction, i.e. lumière ambiante reçue, en ne gardant que les fréquences inférieures à la fréquence de modulation de la source lumineuse, ce qui permet une optimisation de la plage de gain d'amplification utilisable sans risque de saturation ainsi qu'un ratio signal sur bruit beaucoup plus favorable.

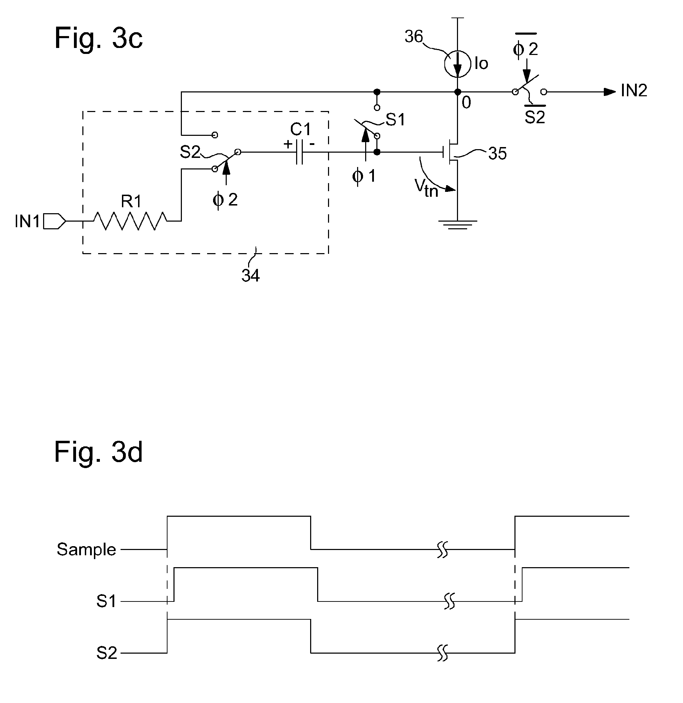

Le circuit de conditionnement 10 comprend ensuite un second étage 16 appelé échantillonneur bloqueur permettant de démoduler le signal reçu IN1 en sortie du premier étage 13. Cet étage échantillonneur bloqueur 16 a pour fonction de rendre le signal utile IN1 continu avant de le fournir en entrée de l'étage suivant. Selon une variante avantageuse de réalisation de ce deuxième étage échantillonneur bloqueur 16 qui sera détaillée en relation avec la figure 3b, il est prévu d'utiliser un circuit échantillonneur bloqueur 17 comprenant des moyens de double échantillonnage corrélé pour démoduler le signal utile. Ces moyens de double échantillonnage corrélé présentent l'avantage de s'affranchir de l'erreur d'offset et du bruit en 1/f. Selon une autre variante avantageuse de réalisation de ce deuxième étage échantillonneur bloqueur 17 qui sera détaillée en relation avec la figure 3c, il est prévu d'utiliser un circuit échantillonneur bloqueur comprenant des moyens de double échantillonnage corrélé associés à un seul transistor actif à la place d'un amplificateur suiveur, ce qui permet de réduire la consommation par rapport à une solution selon la première variante susmentionnée.

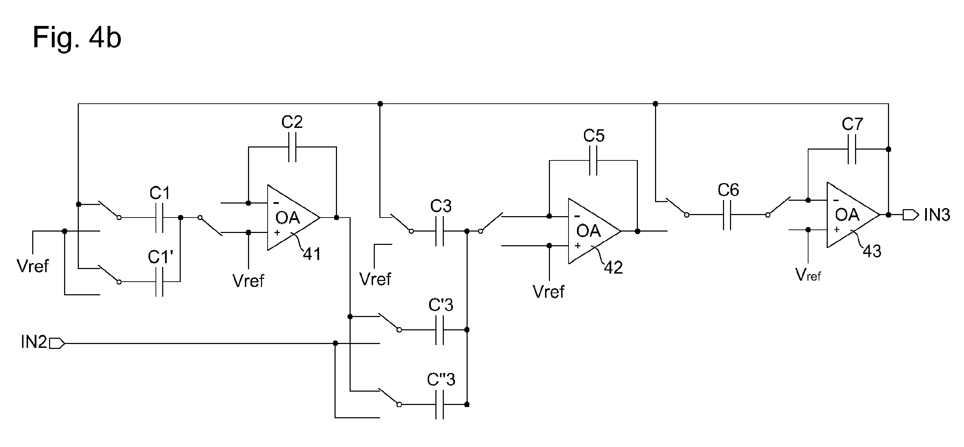

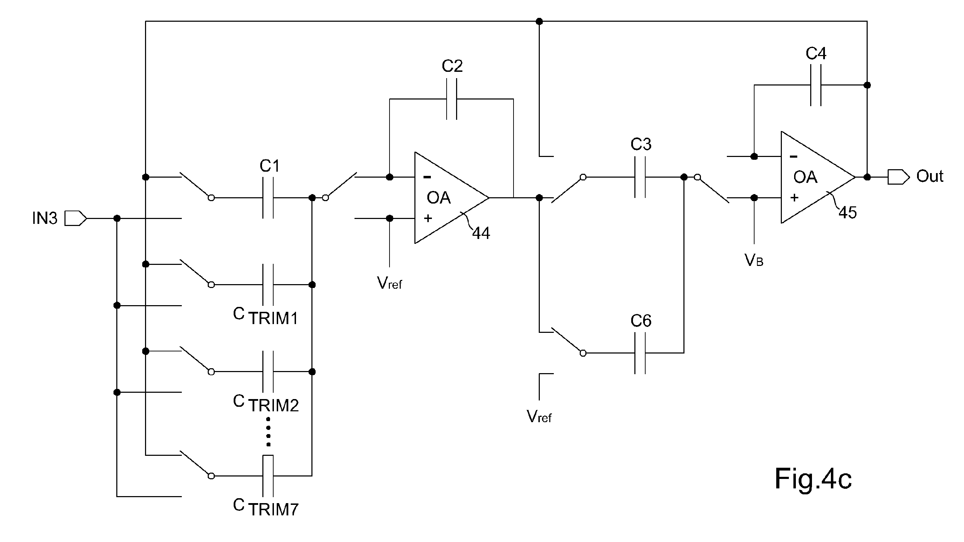

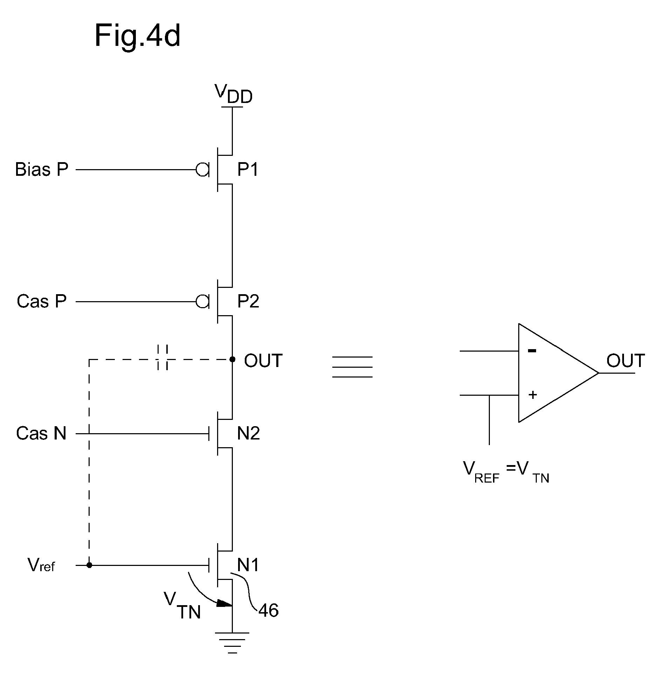

Le circuit de conditionnement comprend encore un troisième étage 18 de filtrage passe-bande au moins du 5ème ordre permettant de filtrer le signal IN2 transmis en sortie du deuxième étage 16 dans la bande de fréquence du signal utile recherchée. Selon une variante de réalisation, il est prévu d'utiliser un circuit à capacités commutées pour réaliser un filtre 19 passe-bande de Bessel du 5ème ordre pour des raisons de réactivité. Ce filtre d'ordre 5 comprend un filtre passe-bande du troisième ordre suivi d'un filtre passe-bas du deuxième ordre, dont les détails seront donnés en relation respectivement avec les figures 4b et 4c. Selon une autre variante avantageuse de réalisation qui sera détaillée en relation avec la figure 4d, le filtre passe bande n'utilise qu'un seul transistor actif au lieu d'un amplificateur opérationnel inverseur pour chacun des quatre premiers étages du filtre, ce qui permet de réduire la consommation ainsi que de s'affranchir du bruit en 1/f. Ainsi dans le filtre du 5ème ordre selon cette autre variante avantageuse de réalisation, seul le dernier étage est réalisé avec un amplificateur opérationnel pour des contraintes de résistance de sortie, de compatibilité avec le convertisseur analogique numérique des moyens de traitement ainsi que d'excursion de tension du signal de sortie. De plus la tension de référence de ce dernier étage est imposée par celle du convertisseur analogique numérique de l'unité de traitement, i.e. le microcontrôleur, tandis qu'avantageusement celle des étages précédents est uniquement dépendante des transistors actifs utilisés. On notera encore que le gain des capacités commutées est ajustable en modifiant les rapports de capacités.

La figure 2a est un schéma fonctionnel du premier étage du circuit de conditionnement selon un mode de réalisation préféré de l'invention. Le signal externe IN est fourni en entrée d'un convertisseur courant tension 23. Aux bornes du convertisseur 23 est prévu une boucle de rétroaction comprenant en série un filtre passe-bas 24 et un convertisseur tension courant 25. Comme il a été vu précédemment, le signal externe IN comprend une composante ambiante et une composante utile correspondant au signal utile que l'on souhaite détecté et transmettre à l'unité de traitement. La boucle de rétroaction a pour but de séparer les deux composantes du signal externe IN. A cet effet, le filtre passe-bas 24 a pour fonction de filtrer les hautes fréquences et notamment celles supportant la composante utile du signal. Ensuite, le convertisseur tension courant 25 a pour but de retransformer la composante ambiante du signal externe sous forme d'un courant pour être soustraite au signal externe IN reçu. Ainsi le signal reçu en entrée du convertisseur courant tension 23 est un signal ne contenant plus que la composante utile du signal externe IN, il est donc possible d'utiliser un convertisseur courant tension avec un gain beaucoup plus élevé que celui pouvant être utilisé selon un circuit présenté en relation avec la figure 7 sans risque de saturation. A titre d'exemple, le signal externe IN reçu est un courant de l'ordre du microampère, tandis que la composante utile de ce signal est de l'ordre du nanoampère soit environ mille fois moins importante. La boucle de rétroaction prévue ici permet donc de soustraire la composante ambiante qui est de l'ordre du milliampère et donc le convertisseur 23 reçoit uniquement la composante utile du signal externe à amplifier, qui est de l'ordre du nanoampère permettant d'utiliser un gain plus élevé sans augmenter le risque de saturation du convertisseur 23 ou des étage suivants utiliser également en tant qu'amplificateurs. Le signal utile IN1 est transmis en sortie du premier étage pour être fourni à l'entrée du deuxième étage.

La figure 2b est une variante avantageuse d'implémentation du mode de réalisation du premier étage du circuit de conditionnement selon la figure 2a. La conversion courant tension du convertisseur courant tension 23 est assurée par un amplificateur opérationnel OA1 et une résistance de contre-réaction Rac. On connectera de préférence une capacité Cac en parallèle de la résistance Rac pour des raisons de stabilité.

Dans le but de détecter la composante continue ou ambiante du signal externe IN reçu, on ajoute un circuit de contre-réaction avec deux intégrateurs réalisant ainsi une fonction de transfert de deuxième ordre, selon la formule suivante:

$$\frac{V_{IN1}}{I_{IN}} = Rac \times \frac{s^2}{s^2 + (\omega_0/Q)s + \omega_0{}^2}$$

Une manière pour implémenter une fonction équivalente de transfert revient à placer un filtre passe-bas à un amplificateur, appelé encore filtre de Sallen et Key, correspondant à un filtre RC passif d'ordre 2 (non représenté) et à un amplificateur opérationnel. Selon une variante avantageuse d'implémentation, tous les composants du filtre passe-bas du deuxième ordre ont été intégrés. A cet effet, les résistances du filtre RC passif sont intégrées sous la forme d'amplificateur opérationnel de transconductance (OTA1, OTA2).

Selon une autre variante, il est possible de prévoir de moyens d'ajustement du gain des amplificateurs opérationnels (OA1, OA2), par exemple aux moyens de résistance Rac et de capacité Cac variables pour l'amplificateur OA1, et une capacité variable C1 pour l'amplificateur OA2. L'ajustement du gain peut être programmé notamment en fonction de l'utilisateur porteur d'un instrument électronique portable comprenant un tel circuit de conditionnement. Les critères d'ajustement peuvent être par exemple dépendants de la circulation sanguine de l'utilisateur ainsi que du niveau d'effort de ce dernier. Si les signaux externes reçus sont puissants le gain pourra être diminué, inversement si les signaux externes reçus sont de faible puissance, le gain pourra être augmenté.

La résistance Rdc assure la conversion de la tension de la composante ambiante en courant qui est soustraite directement à l'entrée sur laquelle est reçu le signal externe IN sous forme d'un courant.

Nous allons maintenant considérer plus en détail des variantes de réalisation du deuxième étage du circuit de conditionnement représenté à la figure 1. Le circuit échantillonneur bloqueur fonctionne comme démodulateur, pour cela ce circuit échantillonne le signal IN1 transmis en sortie du premier étage durant la deuxième partie des impulsions d'échantillonnage et stocke un échantillon entre deux impulsions. En ayant une constante de temps beaucoup plus importante pour le suivi du signal que le temps d'échantillonnage, ce circuit intègre également un filtre anti-repliement de spectre, i.e. un filtre passe-bas éliminant les fréquences supérieures du signal analogique à numériser pour éviter le repliement du spectre. Ce filtre passe-bas auto-intégré assure aussi une fonction de suppression du bruit.

La figure 3a représente un schéma équivalent d'un circuit échantillonneur bloqueur avec filtre intégré utilisable dans le circuit de conditionnement selon l'invention. Ce circuit comprend un filtre passe-bas qui est toujours actif pendant la phase d'échantillonnage. Ce filtre passe-bas permet de supprimer le bruit et fonctionne comme filtre anti-repliement de spectre. Cela peut être réalisé en utilisant une constante de temps qui est beaucoup plus importante que le temps d'échantillonnage. Ainsi, le circuit a une vitesse de balayage limitée. La fréquence du filtre dépend du rapport entre le temps d'échantillonnage et la constante de temps du suivi. Pour une application de détection du rythme cardiaque ou du taux d'oxygène dans le sang, on pourra par exemple envisager un filtre de fréquence à 5 Hz. Le circuit équivalent de ce circuit échantillonneur bloqueur comprend un filtre RC 31, un interrupteur d'échantillonnage 32 étant prévu entre la résistance R et la capacité C du filtre 31. Un amplificateur opérationnel 33 est prévu en sortie du filtre 31 avant transmission du signal échantillonné et bloqué IN2 au troisième étage du circuit de conditionnement.

La figure 3b représente un circuit échantillonneur bloqueur du type à double échantillonnage corrélé, qui permet de s'affranchir en particulier de l'erreur d'offset et du bruit en 1/f. On retrouve comme à la figure 3a, un filtre RC 31 et un amplificateur opérationnel 33. En revanche, l'interrupteur d'échantillonnage 32 a été remplacé par trois interrupteurs d'échantillonnage (deux S1, S2), dont les signaux de commande (Φ1, Φ2) sont donnés à la figure 3d. Le premier interrupteur S1 est agencé entre la résistance R et la capacité C du filtre 31. L'interrupteur S2 est agencé entre le premier interrupteur S1 et la capacité C , d'une part, et la sortie de l'amplificateur opérationnel 33, d'autre part. Enfin, le deuxième interrupteur S1 est agencé entre l'entrée négative et la sortie de l'amplificateur opérationnel 33.

La figure 3c représente un exemple d'implémentation d'un circuit échantillonneur bloqueur à double échantillonnage corrélé utilisant un seul transistor actif en lieu et place de l'amplificateur opérationnel 33 de la figure 3b, ce qui permet en sus de la compensation des erreurs d'offset et du bruit en 1/f, de réduire de manière significative la consommation de courant du deuxième étage.

Dans cet exemple de réalisation d'un circuit échantillonneur bloqueur à double échantillonnage corrélé, le filtre passe-bas 34 est réalisé par un filtre R1C1, trois interrupteurs $S$1, $S$2 et $\overline{S2}$ dont les phases (Φ 1, Φ 2) d'actionnement sont représentées à la figure 3d, un transistor actif 35 remplaçant l'amplificateur opérationnel du schéma de principe de la figure 3b, et une source de courant de polarisation 36. Le transistor actif 35 est connecté de sorte que sa grille, i.e. la borne de commande est contrôlée par la sortie du filtre passe-bas 34, et ses deux bornes de courant sont connectées, pour l'une, à la source de courant de polarisation, et pour l'autre à un potentiel de référence, par exemple la masse du circuit.

Lors de la première phase, le signal d'échantillonnage SAMPLE est par exemple à un niveau haut, les signaux de commande des interrupteurs S1 et S2 étant tous deux également à un niveau, on notera néanmoins que le signal de commande S1 est légèrement en retard par rapport au signal de commande de S2, l'interrupteur S1 étant ainsi fermé, i.e. conducteur, légèrement après l'interrupteur S2 laissant ainsi le temps à la capacité C1 de se charger. Pendant cette première phase, S1 et S2 sont donc fermés, i.e. conducteurs, la capacité C1 est donc chargée par le signal IN1 reçu en entrée de ce deuxième étage transmis en sortie du premier étage moins la tension de seuil du transistor actif 35, soit $V_{C1} = V_{IN1} - V_{TN}$. L'interrupteur $\overline{S2}$ étant dans l'état opposé à l'interrupteur S2, il est pendant cette première phase ouvert, i.e. non conducteur, et le potentiel à la sortie de ce deuxième étage, est déconnecté d'avec le troisième étage et est égal à la tension à la tension de ce seuil du transistor actif $V_0 = V_{TN}$.

On notera que dans cet exemple, représenté à la figure 3c, le transistor actif 35 est un transistor de type NMOS, il est cependant tout à fait envisageable d'utiliser à la place un transistor PMOS, voire un transistor en technologie bipolaire en adaptant adéquatement le circuit d'échantillonnage blocage.

Durant la deuxième phase, le signal d'échantillonnage est à un niveau bas, ainsi que les signaux de commande des interrupteurs S1 et S2. Ainsi les deux interrupteurs S1 et S2 sont alors en position ouverte, i.e. non conducteur, tandis que l'interrupteur $\overline{S2}$ est quant à lui en position fermée, i.e. conducteur. La capacité C1 est flottante à l'une de ses bornes, l'autre étant alors connectée au transistor actif 35, la tension aux bornes de la capacité C1 étant la tension d'entrée. En effet, l'offset introduit par la tension de seuil du transistor actif 35 est supprimé par ce procédé de commande des interrupteurs S1 et S2. La tension de sortie $V_{IN2}$ est quand à elle reliée d'une part à celle de l'entrée du deuxième étage et d'autre part à l'entrée du troisième étage. Le bruit en 1/f du transistor actif 35 est compensé, notamment en raison de la basse fréquence du signal.

Du fait que le signal utile IN2 est disponible à la sortie du deuxième étage uniquement durant une des deux phases (φ2), le circuit de filtrage du troisième étage est cadencé sur les flancs descendants des signaux d'horloge.

Le troisième étage du circuit de conditionnement selon l'invention comprend un circuit passe-bande qui permet de supprimer toutes les fréquences qui ne sont pas dans la bande de fréquences correspondant à l'application. Dans l'exemple de la mesure du rythme cardiaque, le filtre passe-bande peut ainsi être choisi pour ne garder que les fréquences entre 1 Hz et 5Hz. Ce filtre passe-bande est réalisé avantageusement en utilisant la technique des capacités commutées. Dans le cadre de la présente invention, il a été mis en évidence la nécessité d'utiliser un filtre au moins du cinquième ordre et de préférence un filtre de Bessel du cinquième ordre dans la mesure où un filtre du septième ordre bien que plus précis, est trop lent et coûteux en terme de place et de consommation.

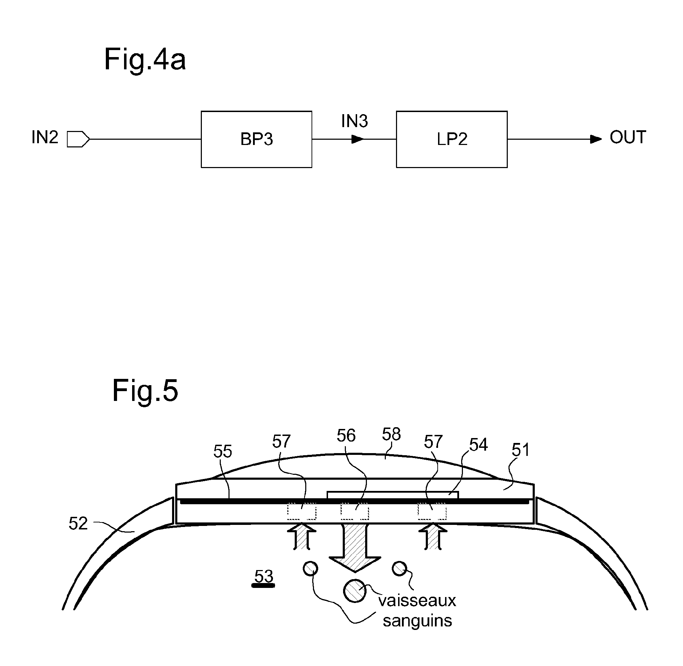

Comme cela est représenté schématiquement à la figure 4a, le filtre passe-bande du cinquième ordre est avantageusement décomposé en deux sections, une première section comprenant un filtre passe-bande du troisième ordre BP3 et une deuxième section comprenant un filtre passe-bas du deuxième ordre LP2. En raison de la haute composante continue du signal d'entrée IN2, le gain de ce filtre doit être après l'étage passe-haut du filtre passe-bande d'ordre 3.

L'impédance de sortie du troisième étage doit être adaptée au convertisseur analogique numérique de l'unité de traitement recevant le signal OUT conditionné. Cette adaptation d'impédance dépend en particulier des contraintes imposées par le convertisseur analogique numérique. C'est pourquoi, selon cette définition, l'impédance de sortie de la source doit être réelle à la fréquence d'entrée et polariser à la tension de référence du convertisseur analogique numérique, soit à une tension $V_B$, par exemple égale à $V_{REF}/2$.

La figure 4b représente un exemple de réalisation de la première section du troisième étage comprenant le filtre passe-bande d'ordre 3. La réalisation de ce filtre passe-bande est basée sur la technique des capacités commutées. Le filtre nécessite l'utilisation de trois amplificateurs opérationnels 41, 42 et 43 précédés de capacités commutables. Considérant l'un des étages de ce filtre passe-bande du troisième ordre, il est avantageusement prévu de pouvoir programmer le gain en ajustant le rapport des capacités, par exemple en ajoutant des capacités d'entrée additionnelles C'1, respectivement C''3) en parallèle des capacités d'entrée (C1, respectivement C'3).

La figure 4c représente un exemple de réalisation de la deuxième section du troisième étage comprenant le filtre passe-bas d'ordre 2. La réalisation de ce filtre passe-bas est également basée sur la technique des capacités commutées. Le filtre nécessite deux amplificateurs opérationnels 44 et 45, le dernier étant en outre utilisé pour l'adaptation d'impédance avec le convertisseur analogique numérique de l'unité de traitement placé en sortie du circuit de conditionnement. Là encore, il est avantageusement prévu d'avoir un gain ajustable en jouant sur le rapport des capacités $C1/C_{TRIM1}.../C_{TRIM7}$ placés en entrée du premier étage du filtre comprenant l'amplificateur opérationnel 44.

Selon un mode de réalisation particulièrement avantageux d'un filtre passe-bande de type Bessel d'ordre 5 tel que présenté en relation avec les figures 4b et 4c, il est prévu de remplacer les quatre premiers amplificateurs opérationnels 41, 42, 43 et 44 par quatre transistors actifs 46, ceci ayant pour effet de réduire drastiquement le courant de consommation du filtre et la contribution au bruit global du circuit. Un des étages de remplacement est représenté à la figure 4d, dans lequel le transistor actif N1 46 remplace un amplificateur opérationnel. La tension de référence $V_{REF}$ est la tension de seuil $V_{TN}$ du transistor. Les transistors P1, P2 et N2 formant la sortie d'un miroir de courant dont l'étage d'entrée de polarisation est commun à tous les étages d'amplification du filtre et permettant de polariser le transistor actif 46.

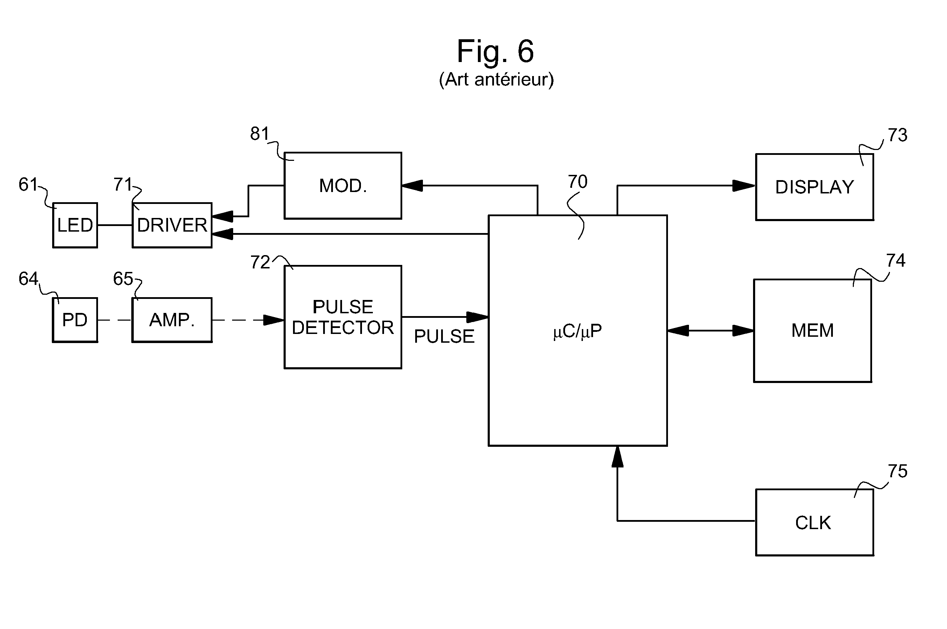

La figure 5 est une vue en coupe d'un instrument électronique portable, comme par exemple une montre-bracelet, comprenant une boîte 51, un bracelet 52 permettant de plaquer la boîte de montre 51 sur le poignet 53 de l'utilisateur. Dans la boîte 51, on retrouve un circuit intégré 54 monté sur une plaquette à circuit imprimé 55. Le circuit intégré 54 comprenant un circuit de conditionnement selon l'un des modes de réalisation de l'invention sus présentés. L'instrument portable comprend encore un dispositif optique 56, 57 qui est amené en permanence au contact du tissu organique de l'utilisateur lorsque l'instrument est porté. Une émission lumineuse est produite par une source 56 agencée pour pénétrer suffisamment profondément dans le tissu organique et pour être modulée par le flux sanguin irriguant le tissu organique illuminé. L'émission lumineuse modulée est détectée après réflexion par des photorécepteurs 57 du dispositif optique. Les signaux détectés sont ensuite conditionnés puis traités respectivement par le circuit de conditionnement et l'unité de traitement intégrés sur le circuit intégré 54. L'instrument portable comprend encore un dispositif d'affichage 58 pour afficher une information relative à la mesure de la grandeur physiologique, notamment le rythme cardiaque. Il est bien entendu également possible à titre d'alternative d'utiliser plusieurs circuits intégrés, notamment un premier circuit intégré pour les moyens d'émission de lumière et un autre circuit intégré pour les moyens de réception comprenant le circuit de conditionnement et l'unité de traitement, ou encore tout autre variante souhaitable.

Avantageusement, il est également prévu sur le circuit intégré 54 comprenant le circuit de conditionnement, de prévoir un circuit de commande programmable de la source lumineuse. Ce circuit de commande programmable est réalisé sous la forme d'une source de courant pour alimenter la source lumineuse 56. La plage de courant peut être ajustée au moyen d'une résistance externe. Le but de ce circuit de commande de la source lumineuse 56 est d'avoir un court temps d'établissement pour générer de courtes impulsions.

Au niveau du circuit intégré, et plus particulièrement du circuit de conditionnement, il est important de disposer de deux tensions de référence, l'une utilisée pour alimenter le convertisseur analogique numérique de l'unité de traitement et l'autre pour polariser le dernier étage du filtre passe-bande ou étage de sortie du troisième étage du circuit de conditionnement. Avantageusement, il est prévu de choisir la tension de polarisation comme une fraction de la tension de référence nécessaire pour l'alimentation du convertisseur analogique numérique. La tension de polarisation pourra par exemple correspondre à la moitié de la tension de référence.

On comprendra que diverses modifications et / ou améliorations et / ou combinaisons évidentes pour l'homme du métier peuvent être apportées aux différents modes de réalisation de l'invention exposés ci-dessus sans sortir du cadre de l'invention défini par les revendications annexées.

**Revendications**

1. Circuit de conditionnement (10) d'un signal externe (IN) représentatif d'une grandeur physiologique entre un capteur optique (11) et une unité de traitement (12), ledit signal externe (IN) reçu étant décomposé en une composante utile et une composante ambiante, **caractérisé en ce que** ledit circuit de conditionnement comprend :

    - un premier étage (13) comprenant un amplificateur de transimpédance avec un filtre passe-haut incorporé utilisant une boucle de rétroaction pour soustraire, en entrée de l'étage, au signal externe reçu, la composante ambiante du signal et délivrer en sortie un signal utile amplifié (IN1);
    - un deuxième étage (16) comprenant un circuit échantillonneur bloqueur (17) pour démoduler ledit signal utile amplifié et délivrant en sortie un signal utile démodulé (IN2); et
    - un troisième étage (18) comprenant un filtre passe-bande (19) pour filtrer ledit signal utile démodulé dans la bande de fréquence de la grandeur physiologique à détecter et pour transmettre un signal conditionné (OUT) à ladite unité de traitement.

2. Circuit de conditionnement (10) selon la revendication 1, dans lequel le premier étage est réalisé au moyen d'un convertisseur courant tension (23), la boucle de rétroaction comprenant un filtre passe-bas (24) et un convertisseur tension courant (25).

3. Circuit de conditionnement (10) selon la revendication 1 ou 2, dans lequel le circuit échantillonneur bloqueur (17) du deuxième étage (16) comprend des moyens de double échantillonnage corrélé (31, 32, 33; 34, 35, 36, S1, S2, $\overline{S2}$) pour démoduler ledit signal utile amplifié (IN1).

4. Circuit de conditionnement (10) selon la revendication 3, dans lequel lesdits moyens de double échantillonnage corrélé sont associés à un amplificateur suiveur comprenant un seul transistor actif (35).

5. Circuit de conditionnement (10) selon l'une des revendications 1 à 4, dans lequel ledit filtre passe-bande (19) dudit troisième étage (18) est un filtre passe-bande d'ordre N, N étant supérieur ou égal à 5.

**6.** Circuit de conditionnement (10) selon la revendication 5, dans lequel ledit filtre passe-bande (19) est un filtre du cinquième ordre de type Bessel réalisé au moyen d'un circuit à capacités commutées comprenant un premier filtre passe-bande du troisième ordre (BP3) en série avec un filtre passe-bas du deuxième ordre (LP2).

**7.** Circuit de conditionnement (10) selon la revendication 5 ou 6, dans lequel chacun des N-1 premiers étages du filtre passe-bande (19) utilise un seul transistor actif (46).

**8.** Circuit de conditionnement (10) selon l'une des revendications 5 à 7, dans lequel le gain dudit filtre passe-bande (19) est ajustable par modification d'au moins un rapport de capacités.

**9.** Circuit intégré (54) comprenant un circuit de conditionnement (10) selon l'une quelconque des revendications précédentes pour recevoir ledit signal externe transmis par ledit capteur optique et délivrant en sortie un signal conditionné (OUT).

**10.** Circuit intégré selon la revendication 9, comprenant en outre une unité de traitement (12) connectée au circuit de conditionnement, recevant ledit signal conditionné et traitant ledit signal pour fournir une information relative au dit signal conditionné.

**11.** Instrument électronique portable (51) au poignet comprenant un circuit intégré (54) selon la revendication 9 ou 10, **caractérisé en ce qu'**il comprend en outre

    - un dispositif optique (56, 57) pour la mesure d'une grandeur physiologique, notamment du rythme cardiaque, ledit dispositif optique comprenant au moins une source lumineuse (56) pour soumettre une portion d'un tissu organique (53) à une émission lumineuse et au moins un capteur optique (57) pour détecter l'intensité de l'émission lumineuse après propagation dans ledit tissu organique;
    - ledit circuit intégré agencé pour traiter les signaux optiques détectés par ledit capteur optique;
    - un dispositif d'affichage (58) pour afficher une information relative à la mesure de la grandeur physiologique, notamment le rythme cardiaque.

Fig.1

Fig. 2a

Fig. 3a

Fig. 3b

Fig. 2b

IN1

C1

OA2

Vpol

OTA2

C2

OTA1

Vpol

Rac

Cac

OA1

Vpol

Rdc

IN

Fig. 3c

Fig. 3d

# Fig.4a

IN2 ▷ ────────→ [ BP3 ] ──IN3──→ [ LP2 ] ────→ OUT

# Fig.5

vaisseaux
sanguins

Fig. 4b

Fig.4c

# Fig.4d

$V_{DD}$

Bias P ——○| P1

Cas P ——○| P2

OUT

$\equiv$

Cas N —| N2

$V_{ref}$ —| N1

$V_{TN}$ 46

OUT

$V_{REF} = V_{TN}$

# Fig. 6
(Art antérieur)

EP 1 832 227 A1

# Fig. 7

**Office européen**

**des brevets**

## RAPPORT DE RECHERCHE EUROPEENNE

Numéro de la demande

EP 06 11 0828

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X<br><br>Y | DE 103 18 764 A1 (REICH, STEFAN)<br>20 novembre 2003 (2003-11-20)<br><br>* figure 1 *<br>* alinéas [0028], [0032], [0034], [0035], [0039], [0040], [0042], [0043] *<br><br>----- | 1<br><br><br>2-11 | INV.<br>A61B5/0245 |
| Y<br><br>A | EP 0 319 159 A (THE BOC GROUP, INC)<br>7 juin 1989 (1989-06-07)<br><br>* figure 1 *<br>* colonne 5, ligne 30 - ligne 47 *<br>* colonne 6, ligne 34 *<br>* colonne 8, ligne 22 - ligne 56 *<br>----- | 2-4,9-11<br><br>1,5-8 | |
| A | US 2003/106989 A1 (BLOEHBAUM FRANK ET AL)<br>12 juin 2003 (2003-06-12)<br>* figure 1 *<br>* alinéas [0031], [0034] *<br>----- | 1,2 | |
| T | ANONYMOUS: "MOSFET" [Online]<br>31 juillet 2006 (2006-07-31), WIKIPEDIA,<br>THE FREE ENCYCLOPEDIA , XP002395825<br>WIKIPEDIA<br>Extrait de l'Internet:<br>URL:http://en.wikipedia.org/wiki/MOSFET><br>[extrait le 2006-08-23]<br>* alinéa [07.2] *<br><br>----- | 3,4 | **DOMAINES TECHNIQUES RECHERCHES (IPC)**<br><br>A61B<br>H04B<br>H01J<br>G08C |
| Y | EP 0 262 779 A (PHYSIO-CONTROL<br>CORPORATION) 6 avril 1988 (1988-04-06)<br>* abrégé; figure 1 *<br>* page 12, ligne 10-12 *<br>* page 12, ligne 32 *<br>* page 12, ligne 40 - ligne 52 *<br>* page 13, ligne 27 - ligne 31 *<br>-----<br>-/-- | 5-8 | |

Le présent rapport a été établi pour toutes les revendications

| Lieu de la recherche | Date d'achèvement de la recherche | Examinateur |
|---|---|---|
| La Haye | 23 août 2006 | Schwenke, Stephanie |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
 
& : membre de la même famille, document correspondant

EPO FORM 1503 03.82 (P04C02)

**Office européen des brevets**

**RAPPORT DE RECHERCHE EUROPEENNE**

Numéro de la demande

EP 06 11 0828

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| T | ANONYMOUS: "Active filter" [Online] 18 juillet 2006 (2006-07-18), WIKIPEDIA, THE FREE ENCYCLOPEDIA , XP002395826 WIKIPEDIA Extrait de l'Internet: URL:http://en.wikipedia.org/wiki/Active_filter> [extrait le 2006-08-23] * phrase 1 - phrase 2 * ----- | 7 | |
| A | EP 0 098 662 A (MOTOROLA, INC) 18 janvier 1984 (1984-01-18) * abrégé * * page 2, ligne 29 - page 3, ligne 17 * ----- | 1-11 | |
| A,D | EP 1 484 009 A (ETA SA MANUFACTURE HORLOGERE SUISSE) 8 décembre 2004 (2004-12-08) * abrégé * * alinéas [0027], [0034], [0036] * ----- | 1-11 | |
| | | | **DOMAINES TECHNIQUES RECHERCHES (IPC)** |

Le présent rapport a été établi pour toutes les revendications

| Lieu de la recherche | Date d'achèvement de la recherche | Examinateur |
|---|---|---|
| La Haye | 23 août 2006 | Schwenke, Stephanie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.** EP 06 11 0828

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-08-2006

| Document brevet cité au rapport de recherche | | Date de publication | Membre(s) de la famille de brevet(s) | | Date de publication |
|---|---|---|---|---|---|
| DE 10318764 | A1 | 20-11-2003 | AUCUN | | |
| EP 0319159 | A | 07-06-1989 | CA | 1324187 C | 09-11-1993 |
| | | | JP | 1259840 A | 17-10-1989 |
| | | | US | 4781195 A | 01-11-1988 |
| US 2003106989 | A1 | 12-06-2003 | AT | 324598 T | 15-05-2006 |
| | | | DE | 10160626 A1 | 18-06-2003 |
| | | | EP | 1319965 A2 | 18-06-2003 |
| EP 0262779 | A | 06-04-1988 | AU | 609411 B2 | 02-05-1991 |
| | | | AU | 7716887 A | 25-02-1988 |
| | | | CA | 1298484 C | 07-04-1992 |
| | | | DE | 3785283 D1 | 13-05-1993 |
| | | | DE | 3785283 T2 | 22-07-1993 |
| | | | JP | 1500495 T | 23-02-1989 |
| | | | WO | 8801150 A1 | 25-02-1988 |
| | | | US | 4913150 A | 03-04-1990 |
| EP 0098662 | A | 18-01-1984 | DE | 3374107 D1 | 19-11-1987 |
| | | | HK | 51690 A | 20-07-1990 |
| | | | JP | 59023642 A | 07-02-1984 |
| EP 1484009 | A | 08-12-2004 | AT | 317664 T | 15-03-2006 |
| | | | DE | 60303536 T2 | 21-09-2006 |

EPO FORM P0460

Pour tout renseignement concernant cette annexe : voir Journal Officiel de l'Office européen des brevets, No.12/82

**EP 1 832 227 A1**

## RÉFÉRENCES CITÉES DANS LA DESCRIPTION

**Documents brevets cités dans la description**

- EP 1484009 A